# EUROPÄISCHE PATENTSCHRIFT

(11) **EP 0 433 642 B1**
(45) Veröffentlichungstag und Bekanntmachung des Hinweises auf die Patenterteilung: **26.02.1997**
(21) Anmeldenummer: 90121455.1
(22) Anmeldetag: 09.11.1990
(51) Int. Cl.: C07D 213/74, G01N 33/18

(54) **Karl-Fischer Reagenz und Verfahren zur Bestimmung von wasser mit Hilfe des Reagenzes.**
Karl-Fischer reagent and process for the determination of water by using the reagent
Réactif de Karl-Fischer et procédé de détermination de présence d'eau à l'aide du réactif

(30) Priorität: 21.11.1989 DE 3938561
(43) Veröffentlichungstag der Anmeldung: 26.06.1991
(73) Patentinhaber: MERCK PATENT GmbH, D-64271 Darmstadt (DE)
(72) Erfinder: Pohlke, Rolf, Dr., W-6109 Mühltal (DE); Fischer, Wolfgang, Dr., W-6100 Darmstadt (DE)

(56) Entgegenhaltungen:
- DE-A- 2 834 605
- DE-A- 3 614 135
- US-A- 2 967 155

## Beschreibung

Die Erfindung betrifft ein Karl-Fischer-Reagenz zur Bestimmung von Wasser, das neben Schwefeldioxid und Jod eine Alkylenbis(2-pyridylamin)-Verbindung der allgemeinen Formel I 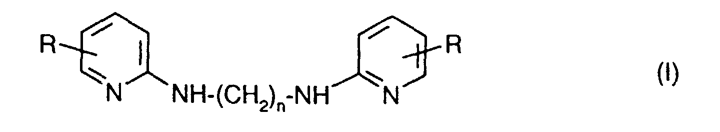 enthält, worin R = H, C₁-C₆-Alkyl und n = 2-8 bedeuten sowie ein Verfahren zur Bestimmung von Wasser mit Hilfe dieses Reagenzes.

Aus DE-A 3 614 135 sind Karl-Fischer-Reagenzien bekannt, die neben Schwefeldioxid und Jod ein Amin enthalten. Als Amine sind auch Verbindungen erwähnt, die zwei Pyridinreste enthalten. Versuche haben gezeigt, daß solche Karl-Fischer-Lösungen, z.B. mit 1,3-Di-(4-pyridyl)propan gegenüber den erfindungsgemäßen Lösungen instabil sind und bereits nach kurzer Zeit Niederschläge auftreten.

Ein Herstellungsverfahren für Verbindungen der allgemeinen Formel ist aus J. Chem. Soc. 1938, 1191-1193, bekannt. Danach werden Alkylenbis(2-pyridylamin)-Verbindungen durch Umsetzung von 2-Aminopyridin mit Dibromalkanen in Gegenwart von Natriumamid in Toluol als Lösungsmittel hergestellt.

Der Erfindung liegt die Aufgabe zugrunde, ein neues Karl-Fischer-Reagenz zur Verfügung zu stellen, das die Nachteile der bekannten Reagenzien nicht besitzt. Diese Aufgabe wurde durch die Bereitstellung des neuen Reagenzes gelöst.

Gegenstand der Erfindung ist ein Karl-Fischer-Reagenz zur Bestimmung von Wasser, das dadurch gekennzeichnet ist, daß es neben Schwefeldioxid und Jod eine Verbindung der allgemeinen Formel I 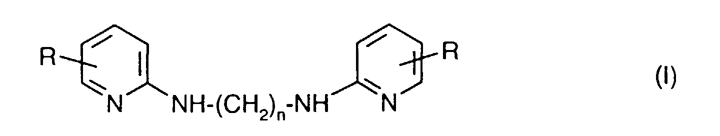 enthält,
worin R = H, C₁ bis C₆-Alkyl und n = 2 bis 8 bedeuten.

Vorzugsweise enthält das Karl-Fischer-Reagenz neben Schwefeldioxid und Jod Ethylenbis(2-pyridylamin).

Ein weiterer Gegenstand der Erfindung betrifft ein Verfahren zur Bestimmung von Wasser mit Hilfe eines Karl-Fischer-Reagenzes, enthaltend eine Verbindung der allgemeinen Formel I, Schwefeldioxid und Jod.

Als Alkylgruppen kommen geradkettige oder verzweigte niedere Alkylgruppen mit 1 bis 6 C-Atomen in Frage, z.B. Methyl, Ethyl, Propyl, i-Propyl, Butyl, i-Butyl, Pentyl, i-Pentyl, Hexyl, i-Hexyl.

Als Alkylene kommen alle geradkettigen oder verzweigten α,ω-Alkylene mit 2 bis 8 C-Atomen in Frage, vorzugsweise solche mit 2 bis 6 C-Atomen, wie Ethylen, Propylen, Butylen, Pentylen, Hexylen und deren Isomeren.

Die Verbindungen der Formel I können mit Erfolg als Ersatzmittel für Pyridin in Karl-Fischer-Reagenzien zur Bestimmung von Wasser eingesetzt werden. Karl-Fischer-Lösungen auf der Basis der erfindungsgemäß hergestellten Verbindungen sind im Vergleich zu Pyridin enthaltenden Lösungen geruchslos. Der Titerabfall dieser Lösungen erfolgt sehr langsam, das heißt sie sind lange haltbar. Die Titrationen mit diesen Karl-Fischer-Lösungen verlaufen schnell und mit stabilem Endpunkt.

### Beispiel

In 1 l Diethylenglycolmonomethylether werden unter Rühren und Feuchtigkeitsausschluß nacheinander
321 g Ethylenbis(2-pyridylamin)
128 g flüssiges Schwefeldioxid und
152 g Jod aufgelöst.

1 ml der Lösung zeigt 5,03 mg Wasser an. Bei der Wasserbestimmung einer Probe werden 20 ml Methanol mit dem obigen Reagenz austitriert. Anschließend wird die Probe zugegeben. Es wird erneut titriert, bis eine schwache Jodfärbng bestehen bleibt. Aus dem Verbrauch der Lösung und der Einwaage der Probe errechnet sich der Wassergehalt.

## Patentansprüche

1. Karl-Fischer-Reagenz zur Bestimmung von Wasser, dadurch gekennzeichnet, daß es neben Schwefeldioxid und Jod eine Verbindung der allgemeinen Formel I 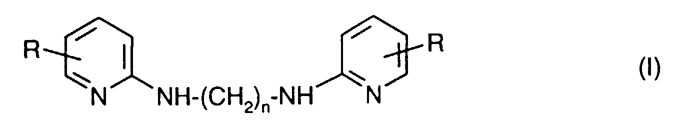 enthält, worin
R = H, C₁ bis C₆-Alkyl und
n = 2-8 bedeuten.

2. Karl-Fischer-Reagenz nach Anspruch 1, dadurch gekennzeichnet, daß es neben Schwefeldioxid und Jod Ethylenbis(2-pyridylamin) enthält.

3. Verfahren zur Bestimmung von Wasser mit Hilfe eine Karl-Fischer-Reagenzes nach den Ansprüchen 1 und 2.

## Claims

1. Karl Fischer reagent for the determination of water, characterized in that, besides sulfur dioxide and iodine, it contains a compound of the general formula I 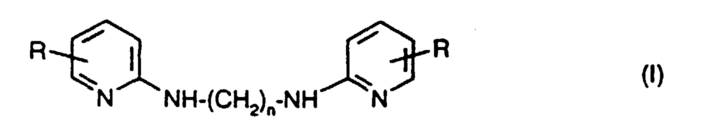 in which
R = H, C₁- to C₆-alkyl and
n = 2-8.

2. Karl Fischer reagent according to Claim 1, characterized in that, besides sulfur dioxide and iodine, it contains ethylenebis(2-pyridylamine).

3. Process for the determination of water with the aid of a Karl Fischer reagent according to Claims 1 and 2.

## Revendications

1. Réactif de Karl-Fischer pour la détermination de l'eau, caractérisé en ce qu'il contient, outre l'anhydride sulfureux et l'iode, un composé de formule générale I 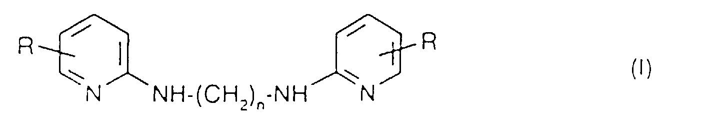 dans laquelle
R = H, un alkyle en C₁ à C₆ et
n = 2 - 8 .

2. Réactif de Karl-Fischer selon la revendication 1, caractérisé en ce qu'il contient, outre l'anhydride sulfureux et l'iode, de l'éthylènebis(2-pyridylamine).

3. Procédé de détermination de l'eau à l'aide d'un réactif de Karl-Fischer selon les revendications 1 et 2.
